# EUROPÄISCHE PATENTSCHRIFT

(11) **EP 2 932 995 B1**
(45) Veröffentlichungstag und Bekanntmachung des Hinweises auf die Patenterteilung: **05.10.2016**
(21) Anmeldenummer: 15158749.0
(22) Anmeldetag: 12.03.2015
(51) Int. Cl.: A61M 13/00, A61B 1/00

(54) **INSUFFLATIONSSYSTEM**
INSUFFLATION SYSTEM
SYSTÈME D'INSUFFLATION

(30) Priorität: 21.03.2014 DE 102014103941
(43) Veröffentlichungstag der Anmeldung: 21.10.2015
(73) Patentinhaber: Storz Endoskop Produktions GmbH, 78532 Tuttlingen (DE)
(72) Erfinder: Lampert, Hugo, 8253 Diessenhofen (CH); Pilatus, Fabian, 78462 Konstanz (DE); Ruh, Peter, 78244 Gottmadingen (DE)
(74) Vertreter: Witte, Weller & Partner Patentanwälte mbB

(56) Entgegenhaltungen:
- WO-A1-2008/122969
- DE-A1-102009 048 994
- US-A- 4 800 869

## Beschreibung

Die Erfindung betrifft ein Insufflationssystem, mit einem medizinischen Instrument, das einen Kanal aufweist, über den ein Insufflationsgas von proximal nach distal führbar ist, wobei das Instrument ein im Kanal angeordnetes Ventil aufweist, das in einem ersten Schaltzustand ein Abführen des Insufflationsgases an die Umwelt bewirkt und in einem zweiten Schaltzustand ein Durchführen des Insufflationsgases durch das Instrument bewirkt, und mit einem Insufflator zum Bereitstellen und Ausgeben des Insufflationsgases an den Kanal.

Die Erfindung betrifft ferner ein Verfahren zum Betreiben eines Insufflationssystems.

Ein derartiges Insufflationssystem ist aus der WO 2007/050516 A bekannt. Ein medizinisches Instrument mit einem derartigen Ventil ist aus der DE 10 2009 048 994 A1 bekannt.

Insufflationssysteme werden im medizinischen Bereich dazu herangezogen, Hohlräume im Körper aufzublähen, damit endoskopische Vorgänge, wie Inspektionen oder Eingriffe in den Körperhöhlen bei besserer Sicht durchgeführt werden können.

Derartige Inspektionen sind beispielsweise Untersuchungen des gastrointestinalen Traktes, beispielsweise bei Vorsorgeuntersuchungen des End- und Dickdarmbereiches. Ein weiteres weit verbreitetes Anwendungsgebiet ist die Laparoskopie des Bauchraumes.

Ein derartiges Insufflationssystem besteht einerseits aus einem medizinischen Instrument, das in die Körperhöhlen einführbar ist und das einen Kanal aufweist, durch den ein Insufflationsgas von proximal nach distal hindurchführbar ist. Das Insufflationsgas wird von einem Insufflator bereitgestellt, der mit dem proximalen Ende des medizinischen Instrumentes beim Einsatz verbunden ist.

Der Insufflator stellt dem Instrument einen bestimmten Volumenstrom zur Verfügung, der für den jeweiligen Vorgang geeignet ist, d.h. es wird durch den Insufflator eine bestimmte Menge an Insufflationsgas durch das Instrument bewegt. Die sog. Durchflussmenge oder Flow-Rate wird üblicherweise in I/min angegeben. Zugleich ist ein bestimmter Insufflationsdruck einstellbar, um diesem an die jeweiligen anatomischen Gegebenheiten anpassen zu können.

Als Insufflationsgas wurde ausgänglich Luft eingesetzt, wobei festgestellt wurde, dass Luft vom Körper schlecht resorbiert wird, und aufgrund des hohen Stickstoffanteiles der Luft postoperative Wirkungen verursacht werden.

Daher wird Luft nach und nach nicht mehr als Insufflationsgas eingesetzt, sondern andere Gase, insbesondere Kohlendioxid, CO₂, das vom lebenden Körper sehr rasch resorbiert und über die Atmung ausgeschieden werden kann.

Im Sinne der vorliegenden Erfindung betrifft somit der Terminus "Insufflationsgas" solche Gase, die zur Insufflation von Körperhöhlen geeignet sind, jedoch nicht Luft.

Kohlendioxid als Insufflationsgas ist medizinisch wenig problematisch und kann auch bei langanhaltenden Untersuchungen oder Eingriffen in relativ hohen Mengen dem Körper zugeführt werden. Im medizinischen Bereich einsetzbares Kohlendioxid ist allerdings relativ teuer.

Bei einem eigentlichen Insufflationsvorgang, also dem Aufblähen von Körperhöhlen, werden Insufflationsgasmengen von beispielsweise 1-3 I/min mit Drücken von etwa 350 mmHg zugeführt.

Im sog. "Standby"-Modus werden etwa 5 I/min bei einem Druck von etwa 80 mmHg durchgeführt.

Bei dem eingangs genannten Insufflationssystem wird eine Steuerung der verschiedenen Zustände dadurch bewirkt, dass am Instrument ein sogenanntes Bypass-Ventil im Kanal angeordnet ist. Dieses Bypass-Ventil weist einerseits einen Durchgang auf, der im Strompfad des Kanales angeordnet ist. Im Ventil ist andererseits eine Abzweigleitung vorhanden, die zur Umwelt mündet. Diese Abzweigleitung endet in einem Kopf eines von Hand beweglichen Ventiles und die Mündung dieser Abzweigleitung liegt im Bereich des Kopfes.

In einem ersten Schaltzustand des Ventiles ist diese Abzweigleitung offen. Das heißt, vom Insufflator zugeführtes Insufflationsgas strömt durch die Abzweigleitung und tritt daher über das Ventil zur Umwelt aus. Das entspricht dem Standby-Modus.

Das Ventil ist so ausgestaltet, dass durch Auflegen eines Fingers einer Hand einer Bedienungsperson, die das Instrument hält, die Austrittsöffnung der Abzweigleitung verschlossen werden kann. Durch Verschließen der Abzweigleitung ist nur noch der Kanal im Instrument offen. Das Gas muss nun durch einen langen und meist dünnen Strömungskanal bis zum Patienten strömen, daher ist ein größerer Widerstand zu überwinden. Durch den höheren Flusswiderstand erfolgt ein Druckanstieg im System.

Dies entspricht dem zweiten Schaltzustand des Ventiles, in dem es nun möglich ist, das Insufflationsgas vom Insufflator ausschließlich durch den Kanal des Instrumentes hindurchzuführen.

Wird die Austrittsöffnung der Abzweigleitung des Ventiles wieder freigegeben, indem der verschließende Finger weggenommen wird, kann das Insufflationsgas wieder über das Ventil in die Umwelt abgegeben werden. Dabei sinkt der Druck im System wieder ab. Diese Druckunterschiede können zur Erkennung der unterschiedlichen Schaltzustände des Ventils herangezogen werden.

Nachteilig an einem solchen Insufflationssystem ist allerdings, dass das relativ teure Insufflationsgas, insbesondere Kohlendioxid, im ersten Schaltzustand permanent aus dem System über das Ventil im Instrument ausströmt und somit für den eigentlichen Insufflationsvorgang verloren ist. Daher müssen Maßnahmen ergriffen werden, um diese austretenden Kohlendioxidmengen zu reduzieren.

Aus der US 2012/0016293 A ist ein endoskopisches System bekannt, durch das CO₂ als Insufflationsgas von proximal nach distal führbar ist. Das Insufflationsgas CO₂ wird aus einer Vorratsflasche zugeführt. Lässt der Druck des zugeführten Insufflationsgas nach, weil der Vorrat in der Flasche zur Neige geht, kann das über Drucksensoren erfasst werden. Bei Unterschreiten eines Druckschwellwertes wird eine Luftpumpe aktiviert, die Pressluft zuführt, um ein Kollabieren einer durch das Insufflationsgas aufgeblähten Operationshöhle während einer Operation zu vermeiden.

Es ist daher Aufgabe der vorliegenden Erfindung hier Abhilfe zu schaffen und ein Insufflationssystem dahingehend weiterzuentwickeln, dass es ökonomischer und sicherer betrieben werden kann, insbesondere unter Ersparnis von kostspieligem I nsufflationsgas.

Erfindungsgemäß wird die Aufgabe dadurch gelöst, dass ein mit dem Kanal verbundener Drucksensor vorhanden ist, durch den die beiden Schaltzustände des Ventils erfassbar sind, und dass dieser mit einem strömungstechnisch vor dem Ventil angeordneten Umschaltventil verbunden ist, das eingangsseitig sowohl mit einer Quelle an Insufflationsgas als auch mit einer Quelle an Luft verbindbar ist, und das ausgangsseitig mit dem Kanal verbunden ist, und dass eine Steuerung vorgesehen ist, die die vom Drucksensor erfassten Druckwerte verarbeitet und die das Umschaltventil auf Verbindung mit der Quelle an Luft schaltet, wenn der Drucksensor den ersten Schaltzustand des Ventiles detektiert, so dass in dem ersten Schaltzustand Luft anstatt Insufflationsgas dem Instrument zuführbar ist und die das Umschaltventil auf Verbindung mit der Quelle an Insufflationsgas schaltet, wenn der Drucksensor den zweiten Schaltzustand detektiert, so dass im zweiten Schaltzustand Insufflationsgas anstatt Luft dem Instrument zuführbar ist.

Diese Maßnahmen haben den Vorteil, dass in denjenigen Betriebsphasen, in denen sich das Ventil des Instrumentes im ersten Schaltzustand befindet, Luft anstatt Insufflationsgas durch das System gefördert wird, die über das Ventil an die Umwelt abgegeben wird.

In diesem erstem Schaltzustand, der bei einem Eingriff über einen überwiegenden Zeitraum vorherrscht, wird also anstatt teurem und gegebenenfalls problematischem Insufflationsgas nur Luft durch das Ventil hindurchgefördert und in die Umwelt abgegeben. Soll dann ein eigentlicher Insufflationsvorgang durchgeführt werden, wird die Abzweigleitung im Ventil verschlossen und es steigt der Druck im System an, was durch den Drucksensor erfasst wird. Das entsprechende Signal wird der Steuerung zugeführt, die das erfasst und dann das Umschaltventil so schaltet, dass es mit der Quelle an Insufflationsgas verbunden wird, so dass dann im zweiten Schaltzustand des Ventiles Insufflationsgas anstatt Luft durch den Kanal des medizinischen Instrumentes zu dessen distalem Ende strömt.

Somit ist vorteilhaft durch einfache Maßnahmen gewährleistet, dass beim eigentlichen Insufflationsvorgang, fast ausschließlich Insufflationsgas in die Körperhöhle eingeführt wird. In dem ersten Zustand, bei dem über relativ lange Zeit erhebliche Mengen an Gas aus dem Ventil abgeführt werden, wird das System mit Luft durchströmt, die über die Abzweigleitung des Ventiles zur Umwelt strömt. Diese ausströmende Luft kann sich mit der Luft im Operationsbereich vermischen und ist völlig unproblematisch.

In einer weiteren Ausgestaltung der Erfindung sind der Drucksensor, das Umschaltventil und die Steuerung im Insufflator angeordnet.

Diese Maßnahme hat den erheblichen Vorteil, dass die Detektions-, Steuer- und Schaltelemente im Insufflator angeordnet sind, so dass an einem solchen Insufflator auch bereits vorhandene Instrumente angeschlossen werden können. Derartige Instrumente sind auf eine lange Betriebsdauer ausgelegt, sind somit aus entsprechenden hochwertigen Materialien hergestellt und sind entsprechend kostspielig. Die an diesen Instrumenten vorhandene Gerätschaften, also der durch das Instrument hindurchgehende Kanal und das in diesem angeordnete Ventil, das in den ersten und zweiten Schaltzustand bringbar ist, können nun mit den erfindungsgemäßen Bauteilen kombiniert werden. Es ist grundsätzlich möglich zumindest die Bauteile Drucksensor und Umschaltventil auch in einem Instrument anzuordnen. Dazu müsste aber dessen Konstruktion entsprechend geändert werden. Durch Vorsehen des Drucksensors und des Umschaltventiles und auch der Steuerung im Insufflator, können bereits vorhandene medizinische Instrumente mit einem erfindungsgemäß aufgerüsteten Insufflator verbunden werden, so dass dann das Gesamtsystem erfindungsgemäß arbeiten kann. Somit ist die Erfindung sehr ökonomisch realisierbar. Der Drucksensor ist immer proximalseitig des Ventils angeordnet. Damit soll der Druck im medizinischen Instrument proximalseitig des Ventils erfassbar sein.

In einer weiteren Ausgestaltung der Erfindung weist die Quelle an Luft einen Kompressor auf, der Umgebungsluft ansaugt und dem Kanal des Instrumentes zuspeist.

Diese Maßnahme hat den erheblichen Vorteil, dass Umgebungsluft herangezogen wird, die in Operationsräumen in ausreichender Menge und auch in einer medizinisch unbedenklichen Qualität zur Verfügung steht. Der Kompressor saugt diese Umgebungsluft an und bringt diese auf den geeigneten Druck und fördert diese mit der gewünschten Durchflussrate durch das Instrument. Das führt ebenfalls zu einer äußerst ökonomischen und auch ergonomischen Realisierung der Erfindung. In Krankenhäusern sind auch Versorgungsleitungen für Luft vorhanden, zum Beispiel für Beatmungsvorgänge, an die die Quelle an Luft anschließbar sein kann.

In einer weiteren Ausgestaltung der Erfindung ist der Kompressor im Insufflator angeordnet.

Diese Maßnahme hat den Vorteil, dass ein besonders kompaktes Gerät zur Verfügung gestellt werden kann, so dass keine baulich ausladenden Maßnahmen am Einsatzort notwendig sind.

In einer weiteren Ausgestaltung der Erfindung weist das Ventil des Instrumentes eine zur Umwelt offene Abzweigleitung auf, die mit dem Kanal verbunden ist.

Diese an sich bekannte Maßnahme hat in Kombination mit der Erfindung den Vorteil, dass die Steuerung zwischen den Schaltzuständen sehr einfach durchführbar ist und die damit verbundenen Druckunterschiede durch den Drucksensor durch konstruktiv einfache Maßnahmen erfassbar sind, in dem eben dieser Drucksensor im Kanal angeordnet ist und diese Druckunterschiede erfassen kann.

In einer weiteren Ausgestaltung der Erfindung weist die Abzweigleitung eine Auslassöffnung zur Umwelt auf, die durch den Finger einer Handhabungsperson verschließbar ist.

Auch diese an sich bekannte Maßnahme stellt in Kombination mit den erfinderischen Maßnahmen ein einfaches Umschalten zwischen dem ersten und zweiten Schaltzustand sicher, der durch den Drucksensor detektiert wird und über die Steuerung in ein Umschalten des Umschaltventiles umsetzbar ist.

In einer weiteren Ausgestaltung der Erfindung ist das Ventil des Instrumentes in einen Schaltzustand bringbar, in dem ein Durchtritt von Insufflationsgas bzw. Luft gesperrt ist, ein weiterer Durchgang des Ventils aber geöffnet ist, und wobei der Strom an Luft in ein Vorratsgefäß mit einer Spülflüssigkeit führbar ist, und der weitere Durchgang mit einem Auslass des Vorratsgefäßes verbunden ist, so dass im dritten Schaltzustand des Ventiles Spülflüssigkeit durch das Instrument von proximal nach distal über die Luft förderbar ist.

Es ist zwar an sich schon bekannt, ein solches Ventil in den dritten Schaltzustand zu bringen, gemäß der Erfindung kann aber nunmehr Luft herangezogen werden, um die Spülflüssigkeit zu fördern und nicht das teure Insufflationsgas. Auch diese Kombination führt zu einer besonders ökonomischen Betriebsweise des Insufflationssystemes.

In einer weiteren Ausgestaltung der Erfindung weist die Steuerung eine Hystereseschaltung auf, die ein Umschalten des Umschaltventils beim Übergang vom ersten Schaltzustand in den zweiten Schaltzustand beim vom Drucksensor erfasstem Druckabfall verzögert.

Diese Maßnahme trägt der Gewohnheit von Ärzten Rechnung, zu Beginn eines Eingriffes mehrere Insufflationsvorgänge kurz hintereinander durchzuführen, um die Körperhöhle nach und nach auf ein bestimmtes Maß aufzublähen. Da diese Insufflationsvorgänge im Abstand einiger Sekunden stattfinden, sorgt diese Zeitverzögerung dafür, dass keine kurzfristigen Umschaltvorgänge zwischen Zuführung von Insufflationsgas und Zuführung von Luft stattfinden müssen, die nach ein oder zwei Sekunden wieder beendet wären.

Nach dem Erzielen eines gewissen Blähzustandes, wird das Gerät oftmals längere Zeit im ersten Schaltzustand des Ventiles betrieben und nur ab und zu, beispielsweise bei schlechter Sicht in der Körperhöhle, oder bei engen Verhältnissen wird ein erneuter Insufflationsvorgang durchgeführt.

Das erfindungsgemäße Insufflationssystem wird daher im Prinzip durch die folgenden Schritte betrieben, nämlich:
Erfassen des Schaltzustandes des Ventiles durch einen Drucksensor,
Zuführen der erfassten Drucksignale zu einer Steuerung,
Schalten des Umschaltventiles durch die Steuerung auf Zufuhr von Insufflationsgas, wenn der zweite Schaltzustand des Ventiles erfasst wird, und
Schalten des Umschaltventiles auf Zufuhr von Luft zum Ventil wenn dessen erster Schaltzustand detektiert wird.

Im praktischen Einsatz ist es vorteilhaft, das Umschalten des Umschaltventiles zeitverzögert durchzuführen.

Besonders ökonomisch kann das Verfahren dadurch durchgeführt werden, dass die zuzuführende Luft im Insufflator komprimiert wird.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend in Zusammenhang mit den beiliegenden Zeichnungen näher beschrieben und erläutert. Es zeigen:
- Fig. 1: stark schematisiert die grundsätzlichen Bauelemente eines erfindungsgemäßen Insufflationssystems,
- Fig. 2: das Ventil des Instrumentes, wobei in Fig. 2a der erste Schaltzustand, in Fig. 2b der zweite Schaltzustand und in Fig. 2c der dritte Schaltzustand dargestellt ist,
- Fig. 3: ein Ausführungsbeispiel eines Insufflationssystemes,
- Fig. 4: drei Graphen zur Erläuterung der Betriebsparameter in den unterschiedlichen Betriebszuständen, wobei der erste Graph den Druck gegenüber die Zeit, der zweite Graph den Schaltzustand des Umschaltventiles gegenüber die Zeit und der dritte Graph den Volumenstrom gegenüber der Zeit darstellt, und
- Fig. 5: stark schematisiert ein weiteres Ausführungsbeispiel eines erfindungsgemäßen Insufflationssystems, bei dem ein an sich schon vorhandenes Instrument mit einem Insufflator und einem Spülsystem dargestellt ist.

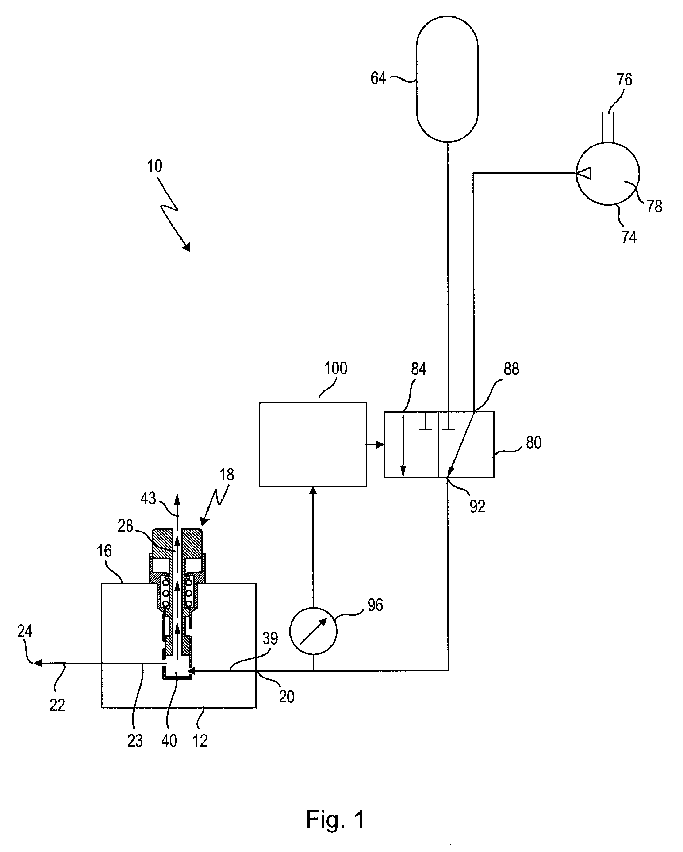

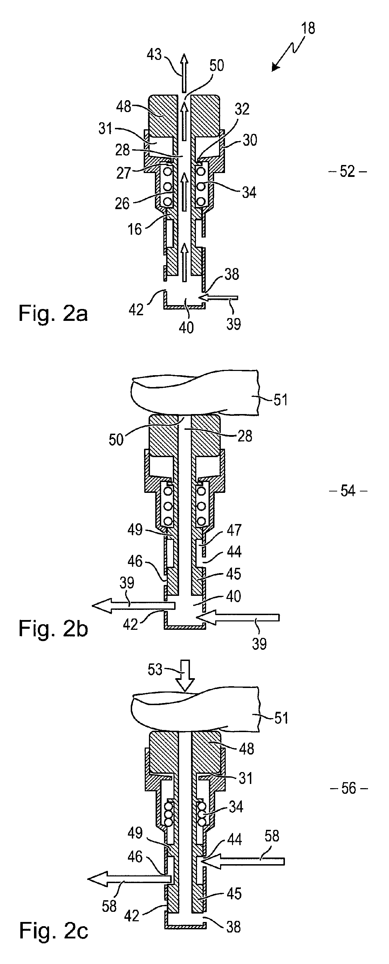

In Zusammenhang mit den Figuren 1 und 2 sollen zunächst die grundsätzlichen Bauelemente des Insufflationssystemes beschrieben werden.

Ein in Fig. 1 dargestelltes Insufflationssystem ist in seiner Gesamtheit mit der Bezugsziffer 10 bezeichnet.

Das Insufflationssystem 10 weist ein medizinisches Instrument 12 auf, das beispielsweise als ein Koloskop ausgebildet ist.

Das medizinische Instrument 12 weist einen Kanal 23 auf, durch den gasförmige oder flüssige Medien von einem proximalseitigen Anschluss 20 zu einem distalseitigen Auslass 24 geführt werden können. Bei einem Koloskop ist ein langer flexibler Schaft 22 vorhanden, der über den After zu Untersuchungen des Darmtraktes einführbar ist.

Mit dem Kanal 23 in Verbindung stehend, ist ein Ventil 18 angeordnet. Die nähere Ausgestaltung und die unterschiedlichen Betriebszustände des Ventiles 18 sollen zunächst in Zusammenhang mit den Figuren 2a bis 2c beschrieben werden.

Das Ventil 18 weist ein Gehäuse 30 auf, in dem ein Ventilkörper 26 hin- und her beweglich aufgenommen ist. Durch den Körper 26 hindurch, reicht über seine Längserstreckung eine Bohrung 28. Der Körper 26 weist an seiner Außenseite einen äußeren Ringflansch 27 auf, der an einer Unterseite eines inneren Ringflansches 32 des Gehäuses 30 anschlagen kann. Eine Feder 34, die in einem Zwischenraum zwischen der Außenseite des Körpers 26 und der Innenseite des Gehäuses 30 angeordnet ist, ist derart vorgespannt, dass sie den Körper 26 in die in Fig. 2a dargestellte Position drückt. Dabei stützt sie sich einerseits auf einem unteren nach innen gerichteten, hier nicht näher bezeichneten Vorsprung des Gehäuses 30 und andererseits an der Unterseite des äußeren Ringflansches 27 des Körpers 26 ab.

Am unteren Ende des Gehäuses 30 ist eine Einlassöffnung 38 vorhanden, der gegenüberliegend eine Auslassöffnung 42 angeordnet ist.

Zwischen den beiden Öffnungen 38 und 42 befindet sich eine Kammer 40.

In dem in Fig. 2a dargestellten Zustand bildet ein unteres Ende des Körpers 26 einen oberen Abschluss der Kammer 40. Das Gehäuse 30 ist dabei derart in das Instrument 12 eingesetzt, dass die Einlassöffnung 38 und die Auslassöffnung 42 mit dem Kanal 23 strömungstechnisch in Verbindung stehen.

Am in der Darstellung von Fig. 2 oberen Ende ist der Körper 26 mit einem radial erweiterten Kopfstück 48 versehen, das in einer entsprechenden Ausnehmung 31 am oberen Ende des Gehäuses 30 aufgenommen ist. Wie zuvor beschrieben, erstreckt sich die Bohrung 28 auch durch das Kopfstück 48 hindurch und endet in einer Auslassöffnung 50.

Wie aus Fig. 2a ersichtlich, ragt das Kopfstück 48 in dem in Fig. 2a dargestellten Betriebszustand etwas über das Gehäuse 30 hinaus.

Dieser Betriebszustand des Ventiles 18 ist ein erster Schaltzustand 52.

In diesem ersten Schaltzustand 52 strömt ein gasförmiges Medium, wie es durch den Pfeil 39 dargestellt ist, über die Einlassöffnung 38 in die Kammer 40 ein. Das zugeführte Gasmedium 39 tritt über die zur Außenseite hin offene Bohrung 28 im Ventilkörper 26 durch das Ventil 18 zur Umwelt aus. Dies deswegen, da der Strömungswiderstand des langen Kanales 23 bis zum Patienten wesentlich größer ist als der Strömungswiderstand in der Bohrung 28. Daher tritt der gesamte Gasstrom 48 über diese Abzweigleitung des Ventils 18 aus.

In Fig. 2b ist eine Situation dargestellt, in der ein Finger 51 auf die Oberseite des Kopfstückes 48 des Ventilkörpers 26 gelegt ist, und zwar derart, dass die Auslassöffnung 50 der Bohrung 28 verschlossen ist.

In diesem Schaltzustand, der einen zweiten Schaltzustand 54 des Ventiles 18 darstellt, strömt nunmehr die dem Ventil 18 zugeführte Gasmenge 39 über die Kammer 40 aus der Auslassöffnung 42 hinaus, also die gesamte zugeführte Gasmenge 39 wird durch das Ventil 18 und den Kanal 23 hindurchgeführt.

In diesem zweiten Schaltzustand 54 wird das Insufflationsgas 39 durch das Ventil 16 hindurch in den Kanal 23 bis zu dessen distalem Ende, also dem Auslass 24 geführt, und zwar mit der gewünschten Durchflussrate und dem gewünschten Druck, beispielsweise mit 1 - 3 I/min durchgeführter Insufflationsgasmenge bei einem Druck von 350 mmHg.

In Fig. 2c ist ein dritter Schaltzustand 56 des Ventiles 18 dargestellt. In diesem Schaltzustand wurde durch den Finger 51 der Handhabungsperson der Ventilkörper 26 gegen die Kraft der Feder 34 "nach unten" bewegt, und zwar so weit, bis sich das Kopfstück 48 maximal weit in die Aussparung 31 im Gehäuse 30 hineinbewegt hat.

In diesem dritten Schaltzustand 56 hat sich ein unterer Kolbenabschnitt 45 des Ventilkörpers 26 vor die Auslassöffnung 42 geschoben.

Dadurch ist es nicht mehr möglich, dass über die Einlassöffnung 38 des Gehäuses 30 Gasmengen durch das Ventil 18 hindurchtreten. Einerseits ist durch das Auflegen des Fingers 51 die Bohrung 28 verschlossen, andererseits ist durch den Kolbenabschnitt 45 die Auslassöffnung 42 verschlossen.

Im dritten Schaltzustand 56 ist eine über der Einlassöffnung 38 angeordnete Spülstromeinlassöffnung 44 geöffnet sowie eine abströmseitige Spülstromauslassöffnung 46. In diesem Bereich weist der Ventilkörper 26 einen geringeren Außendurchmesser als im Bereich des Kolbenabschnittes 45 auf. Dadurch ist eine sog. Spülstromkammer 47 geschaffen, deren oberer Abschluss ein weiterer durchmessergrößerer Kolbenabschnitt 49 des Ventilkörpers 26 bildet.

Wie in Fig. 2c dargestellt, kann nun ein Spülstrom 58 aus einem beispielsweise flüssigen Medium etabliert werden, der über die offene Spülstromeinlassöffnung 44 in die Spülstromkammer 47 eintritt und über die Spülstromauslassöffnung 46 wieder aus dem Ventil 18 austritt. Je nach Ausgestaltung des Instrumentes kann der Spülstrom 58 stromabwärts des Ventiles 18 direkt in den Kanal 23 eintreten oder gegebenenfalls in einen separaten Spülstromkanal. Dadurch kann in dem in Fig. 2c dargestellten dritten Schaltzustand über das Ventil 18 ein Spülstrom von proximal nach distal durch das Instrument 12 hindurchgeführt werden.

Das dem menschlichen oder tierischen Körper zugeführte Spülmedium, meistens eine flüssige Spüllösung, kann dann über eine hier nicht näher dargestellte Absaugleitung des Instrumentes abgesaugt werden.

Wird das Ventil 18 aus dem in Fig. 2c dargestellten Schaltzustand wieder freigegeben, sprich der Finger 51 vollständig vom Kopfstück 48 weggenommen, drückt die Feder 34 den Ventilkörper 26 in den in Fig. 2a dargestellten Schaltzustand 52.

Zurückkehrend nunmehr zu Fig. 1 ist dort das Ventil 18 in dem ersten Schaltzustand 52 dargestellt. Entsprechend der Erfindung ist ein Drucksensor 96 vorgesehen, der strömungstechnisch mit dem Kanal 23 verbunden ist und die darin herrschenden Druckverhältnisse erfasst. Der Drucksensor 96 ist mit einer Steuerung 100 verbunden, die das vom Drucksensor 96 ausgegebene Signal erfasst und verarbeitet.

Des Weiteren ist ein Umschaltventil 80 vorgesehen, das strömungstechnisch über dessen Ausgang 92 ebenfalls mit dem Kanal 23 verbunden ist.

Eingangsseitig ist das Umschaltventil 80 über einen ersten Eingang 84 mit einer Quelle an Insufflationsgas 64 verbunden. In der Praxis ist das eine Gasflasche, oder eine Zentral-/Hausversorgung, die für den medizinischen Bereich aufgearbeitetes und geeignetes komprimiertes Kohlendioxid enthält. Das Umschaltventil 80 weist noch einen zweiten Eingang 88 auf, der mit einer Quelle 78 an Luft verbunden ist. Es ist zu erkennen, dass der Drucksensor 96 proximalseitig, also stromaufwärts, zum Ventil 18 angeordnet ist. Ferner ist der Drucksensor 96 strömungstechnisch zwischen dem Ventil 18 und dem Umschaltventil 80 angeordnet.

Die Quelle an Luft 78 weist einen Kompressor 74 auf, der über einen Ansaugstutzen 76 Luft aus der Umwelt ansaugt und komprimiert. Die Luft kann auch aus einer zentralen Hausversorgung stammen.

Wie aus Fig. 1 zu entnehmen, schaltet die Steuerung 100 das Umschaltventil 80 in einen Betriebszustand, in dem der Eingang 88 des Umschaltventiles 80 mit der Quelle an Luft 78 verbunden ist, sofern sich das Ventil 18 im medizinischen Instrument 12 im ersten Schaltzustand 52 befindet. In diesem Zustand wird, wie zuvor beschrieben, ausschließlich Luft aus der Quelle an Luft 78 über das Umschaltventil 80 dem Anschluss 20 des Instrumentes 12 zugeführt. Über die Bohrung 28 strömt die Luft wieder aus. Wird nun das Ventil 18 in den zweiten in Fig. 2b dargestellten Schaltzustand 54 gebracht, erfolgt ein Druckanstieg in dem Kanal 23. Dieser Druckanstieg kann durch den Drucksensor 96 erfasst werden und gibt ein entsprechendes Drucksignal an die Steuerung 100 weiter.

Erfasst die Steuerung 100 diesen Druckanstieg, wobei aus schaltungstechnischen Gründen hier ein bestimmter Schwellenwert überschritten werden muss, wie das nachfolgend noch beschrieben wird, gibt diese ein Schaltsignal an das Umschaltventil 80 dahingehend, dass dieses auf Durchfluss von Insufflationsgas, sprich Kohlendioxid, schaltet. Durch die Ausgestaltung als Magnetventil wird ein entsprechendes Bauteil, z. B. eine Wippe oder ein Kolben bewegt, das dafür sorgt, dass der erste Eingang 84 mit der Quelle 64 an Insufflationsgas verbunden ist und der dann mit dem Ausgang 92 verbunden ist. In diesem Schaltzustand kann dann keine Luft mehr über den Eingang 88 zum Ausgang 92 strömen. In diesem Schaltzustand kann nun ein Insufflationsvorgang durchgeführt werden.

Das kann beispielsweise mit einer Durchflussrate von 1 - 3 I/min an Insufflationsgas bei einem Druck von 350 mmHg durchgeführt werden.

Nimmt das Ventil 18 wieder seinen ersten Schaltzustand 52 an, wie zuvor beschrieben, ist das mit einem entsprechenden Druckabfall verbunden, der wiederum vom Drucksensor 96 erfasst wird. Dessen Signal wird dann von der Steuerung 100 verarbeitet und dazu herangezogen, das Umschaltventil 80 wieder auf die Durchfuhr von Luft zu schalten, wie das in Fig. 1 dargestellt ist.

Die Bauelemente Drucksensor 96 und Umschaltventil 80 können auch in das Instrument 12 integriert werden. Prinzipiell könnte auch die Steuerung 100 in das medizinische Instrument integriert werden, es müssten dann entsprechende Anschlüsse an die Quelle 64 an Insufflationsgas und die Quelle 78 an Luft vorgesehen werden.

Es ist auch möglich nur den Drucksensor 96 und das Umschaltventil 80 in das Instrument 12 zu integrieren und die Steuerung 100 einem separaten Gerät, nämlich einem Insufflator zuzuordnen.

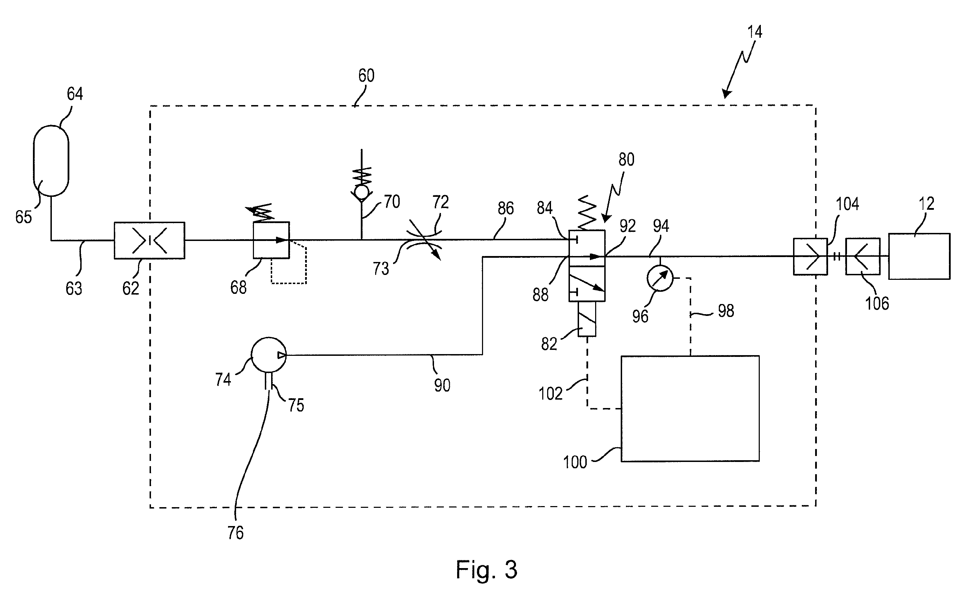

In Fig. 3 ist eine Variante des erfindungsgemäßen Insufflationssystemes 10 dargestellt, bei dem die zuvor beschriebenen Bauteile in einem Insufflator 14 aufgenommen sind, der ausgangsseitig einen Instrumentenanschluss 104 aufweist. An diesen Instrumentenanschluss 104 kann ein entsprechender Anschluss 106 eines Instrumentes 12 angeschlossen werden, das, wie zuvor beschrieben, mit einem Ventil 18 ausgestattet ist. Der in Fig. 3 dargestellte Insufflator 14 weist einen Anschluss 62 auf, mittels dem er über eine Leitung 63 mit einer Quelle 64 an Insufflationsgas verbunden werden kann.

Das kann eine Gasflasche 65 sein, in der das Insufflationsgas, meist Kohlendioxid, unter einem relativ hohen Druck komprimiert aufgenommen ist. In Krankenhäusern ist eine Haus- bzw. Zentralversorgung üblich.

Im Insufflator 14 ist ein Druckregler 68 angeordnet, über den der für den medizinischen Eingriff bzw. der für den Betrieb des medizinischen Instrumentes 12 gewünschte Druck einstellbar ist.

Ein nachgestaltetes Sicherheitsventil 70 sorgt dafür, dass nicht versehentlich bei einer Betriebsstörung des Druckreglers 68 hohe Gasdrücke über das Instrument 12 einem menschlichen Körper zugeführt werden. Nach dem Sicherheitsventil ist ein Flussmengenregler 72 angeordnet, der eine Düse 73 aufweist, über den die jeweiligen Durchflussmengen einstellbar sind. Es können hier auch mehrere parallel geschaltete Gaspfade vorgesehen werden, in denen jeweils Düsen sitzen, um dadurch bestimmte vorgegebene Flussraten zu bewirken. Über eine Leitung 86 ist diese Gasleitung mit dem ersten Eingang 84 eines Umschaltventiles 80 verbunden, das hier als Magnetventil 82 ausgebildet ist.

Im Insufflator 14 ist ferner ein Kompressor 74 angeordnet, der über einen Saugstutzen 75 Umgebungsluft 76 ansaugt und entsprechend komprimiert.

Der Ausgang des Kompressors 74 ist über eine Leitung 90 mit dem zweiten Eingang 88 des Umschaltventiles 80 verbunden.

Das Magnetventil 82 ist über eine Steuerleitung 102 mit einer Steuerung 100 verbunden.

Stromabwärts des Umschaltventiles 80, also nach dessen Ausgang 92, ist in der Leitung 94, die zu dem Instrumentenanschluss 104 führt, ein Drucksensor 96 angeordnet. Dessen Drucksignal wird über eine Steuerleitung 98 der Steuerung 100 zugespeist.

In Fig. 3 ist durch die gestrichelte Linie ein Gehäuse 60 des Insufflators 14 dargestellt, d.h. alle zuvor beschriebenen Bauteile sind im Insufflator aufgenommen. Lediglich die Gasflasche 65 ist außerhalb des Gehäuses 60 angeordnet, so dass diese im Bedarfsfall ausgewechselt werden kann.

Im Betrieb des Insufflationssystemes 10 ist ein medizinisches Instrument 12 über dessen Anschluss 106 mit dem Instrumentenanschluss 104 des Insufflators 14 verbunden.

Die entsprechende Energieversorgung, sprich Stromversorgung, und die entsprechende logische Schaltung in der Steuerung 100 sind hier nicht dargestellt.

Somit ist dann das Insufflationssystem 10 betriebsbereit.

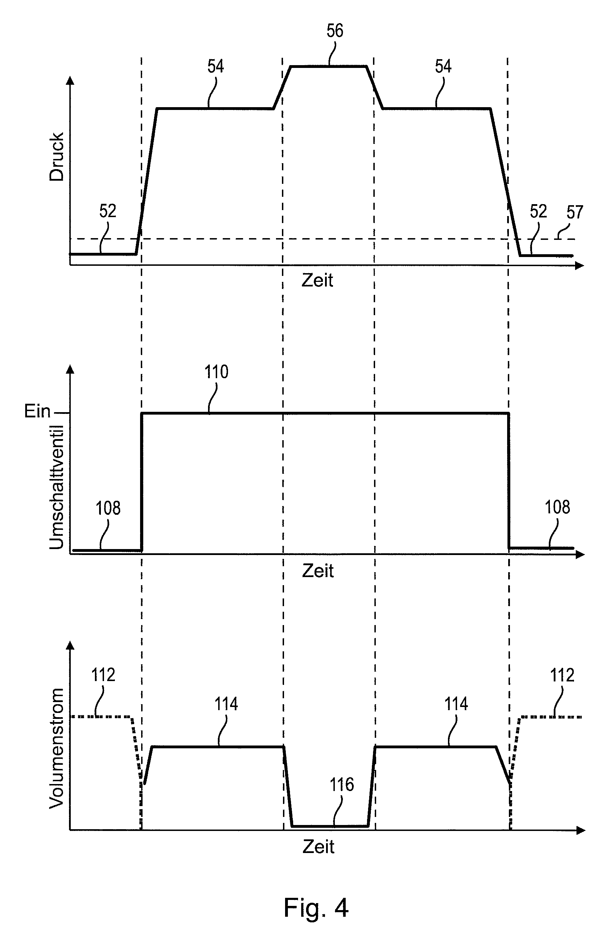

In Zusammenhang mit den Graphen von Fig. 4 soll das Funktionsmuster der unterschiedlichen Betriebszustände erläutert werden. Im obersten Graph ist der Druck im Insufflator gegenüber der Zeit dargestellt. Im ersten Schaltzustand 52 des Ventiles herrscht im Insufflator ein Druck, der unter einer bestimmten Schaltschwelle 57 liegt.

Liegt der Druck bei etwa 80 mmHg, so kann die Schaltschwelle 57 etwa 120 mmHg betragen, um situationsbedingte Druckschwankungen im ersten Schaltzustand 52 des Ventiles unbeachtet zu lassen.

Im zweiten mittleren Graph ist der Schaltzustand des Umschaltventiles 80 gegenüber der Zeit dargestellt, wobei ein Schaltzustand 108 einen Zustand darstellt, in dem kein Insufflationsgas, sprich CO₂, strömt, sondern ausschließlich Luft.

Im untersten Graph in Fig. 4 ist der jeweilige Volumenstrom an Medien, die durch das System hindurchströmen, dargestellt. Die gestrichelte Linie stellt den Luftstrom und die durchgezogene Linie stellt den Insufflationsgasstrom dar.

In dem ersten Schaltzustand 52 des Ventiles 18 und dem Schaltzustand 108 des Umschaltventiles strömt lediglich Luft 112 durch das Instrument.

Wird nun das Ventil 18 auf den zweiten Schaltzustand 54 gebracht, steigt der Druck am Instrumenteneingang deswegen an, da jetzt die gesamte zugeführte Gasmenge durch das Instrument durchgeführt werden muss und nicht mehr über das Ventil entweichen kann. Dieser Druckanstieg wird durch den Drucksensor 96 erfasst und von der Steuerung 100 wird das Umschaltventil 80 in den Schaltzustand 110 geschaltet, d.h. das Umschaltventil 80 ist nun auf ausschließlich Durchfluss von Insufflationsgas, sprich CO₂, geschaltet. Wie aus dem unteren Graph ersichtlich, nimmt dadurch der Volumenstrom an Luft 112 ab und zugleich steigt der Volumenstrom an Insufflationsgas, CO₂, 114 bis auf ein bestimmtes Level an. In diesem Zustand wird ein Insufflationsvorgang durchgeführt.

Wird das Ventil 18 im Instrument nun auf den dritten Schaltzustand 56 gebracht, steigt der Druck weiter an, da nunmehr kein gasförmiges Medium durch das Ventil 18 hindurchtritt, wie das in Zusammenhang mit Fig. 2c beschrieben worden ist.

Das Umschaltventil ist in diesem Zustand weiterhin offen und der Gasstrom, hier der des Insufflationsgases, kann dazu herangezogen werden, eine Spülflüssigkeit durch das Instrument hindurchzuführen. Dadurch sinkt der Volumenstrom entsprechend ab, da dieser lediglich zum Austreiben und Durchführen der Spülflüssigkeit herangezogen wird. Wird das Ventil 18 wieder in den zweiten Betriebszustand 54 verbracht, wird ausschließlich Insufflationsgas 114 durch das Instrument geführt.

Wird das Ventil 18 vollständig freigegeben, also der Finger 51 von der Auslassöffnung 50 abgehoben, fällt der Druck stark ab, dabei unter die Schaltschwelle 57. Dann schaltet das Umschaltventil auf den Zustand 108 um, in dem wieder nur Luft durch das Instrument hindurchgeführt wird, so dass dann der Strom an Luft 112 wieder entsprechend ansteigt.

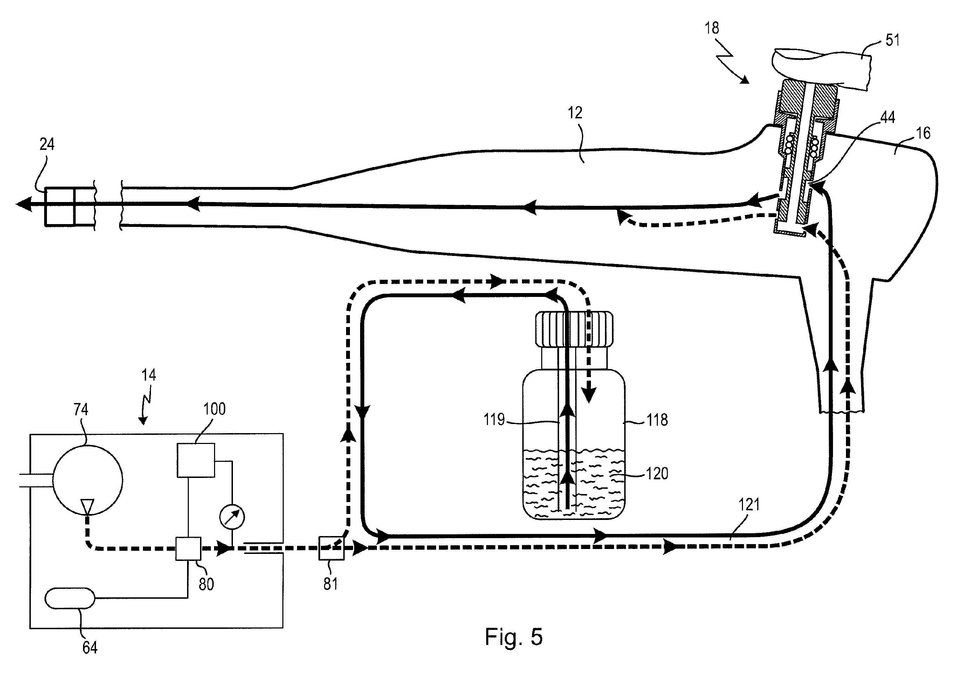

Aus Fig. 5 ist ersichtlich, dass es sich dort um ein konventionelles medizinisches Instrument 12, nämlich ein Koloskop, handelt, das mit einem Ventil 18 versehen ist. In Fig. 5 befindet sich das Ventil 18 in dem dritten Schaltzustand 56. Über eine Weiche 81 kann der vom Insufflator 14 kommende Gasstrom in ein Gefäß 118 eingeleitet werden, in dem eine Spülflüssigkeit 120 aufgenommen ist.

In die Spülflüssigkeit 120 taucht eine Steigleitung 119 ein, die über eine Leitung 121 mit der Spülstromeinlassöffnung 44 des Ventiles 18 verbunden ist.

In diesem Zustand wird dann Spülflüssigkeit 120 durch das Instrument 12 hindurch bis zu dessen distalem Auslass 24 geführt.

Zuvor wurde beschrieben, dass das Insufflationsgas zum Fördern der Spülflüssigkeit herangezogen wird.

Es ist aber prinzipiell auch möglich, anstatt Insufflationsgas Luft aus dem Kompressor 74 zuzuführen.

Das bedeutet, dass man extra eine Schaltlogik vorsieht, die ausschließlich dem Zustand "Spülen" dient. Das bedeutet, befindet sich das Ventil 18 im dritten Schaltzustand 56, wie er in Fig. 2c dargestellt ist, kann die Steuerung 100 das Umschaltventil 80 auf den Zustand schalten, dass lediglich Luft durchgeführt wird, die dann zum Austreiben der Spülflüssigkeit 120 aus dem Gefäß 118 dient.

In Fig. 5 stellt die gestrichelte Linie den Luftstrom dar, die durchgezogene Linie stellt den Strom an Spülflüssigkeit dar.

## Patentansprüche

1. Insufflationssystem, mit einem medizinischen Instrument (12), das einen Kanal (23) aufweist, über den ein Insufflationsgas (39) von proximal nach distal führbar ist, wobei das Instrument (12) ein mit dem Kanal (23) in Verbindung stehendes Ventil (18) aufweist, das in einem ersten Schaltzustand (52) ein Abführen des Insufflationsgases an die Umwelt bewirkt und in einem zweiten Schaltzustand (54) ein Durchführen des Insufflationsgases durch das Instrument (12) bewirkt, und mit einem Insufflator (14) zum Bereitstellen und Ausgeben des Insufflationsgases (39) an den Kanal (23), wobei ein mit dem Kanal (23) verbundener Drucksensor (96) vorhanden ist, durch den die beiden Schaltzustände (52, 54) des Ventiles (18) erfassbar sind, und mit einer Steuerung (100), die die vom Drucksensor (96) erfassten Druckwerte verarbeitet, **dadurch gekennzeichnet, dass** ein strömungstechnisch vor dem Ventil (18) angeordnetes Umschaltventil (80) vorgesehen ist, das eingangsseitig sowohl mit einer Quelle (64) an Insufflationsgas als auch einer Quelle (78) an Luft verbindbar ist, und wobei das Umschaltventil (80) ausgangsseitig mit dem Kanal (23) verbunden ist, und die Steuerung (100) das Umschaltventil (80) auf Verbindung mit der Quelle (78) an Luft (76) schaltet, wenn der Drucksensor (96) den ersten Schaltzustand (52) des Ventiles (18) detektiert, so dass in dem ersten Schaltzustand (52) Luft anstatt Insufflationsgas dem Instrument (12) zuführbar ist, und die das Umschaltventil (80) auf Verbindung mit der Quelle (64) an Insufflationsgas (39) schaltet, wenn der Drucksensor (96) den zweiten Schaltzustand (54) detektiert, so dass im zweiten Schaltzustand (54) Insufflationsgas (39) anstatt Luft (76) dem Instrument (12) zuführbar ist.

2. Insufflationssystem nach Anspruch 1, **dadurch gekennzeichnet, dass** der Drucksensor (96), das Umschaltventil (80) und die Steuerung (100) im Insufflator (14) angeordnet sind.

3. Insufflationssystem nach Anspruch 1 oder 2, **dadurch gekennzeichnet, dass** die Quelle (78) an Luft einen Kompressor (74) aufweist, der Umgebungsluft (76) ansaugt und dem Kanal (23) des Instrumentes (12) zuspeist.

4. Insufflationssystem nach Anspruch 3, **dadurch gekennzeichnet, dass** der Kompressor im Insufflator (14) angeordnet ist.

5. Insufflationssystem nach einem der Ansprüche 1 bis 4, **dadurch gekennzeichnet, dass** das Ventil (18) des Instrumentes (12) eine zur Umwelt offene Abzweigleitung (28) aufweist, die mit dem Kanal (23) verbunden ist.

6. Insufflationssystem nach Anspruch 5, **dadurch gekennzeichnet, dass** die Abzweigleitung (28) eine Auslassöffnung (50) zur Umwelt aufweist, die durch den Finger (51) einer Handhabungsperson verschließbar ist.

7. Insufflationssystem nach einem der Ansprüche 1 bis 6, **dadurch gekennzeichnet, dass** das Ventil (18) des Instrumentes (12) in einen dritten Schaltzustand (56) bringbar ist, in dem ein Durchtritt von Insufflationsgas bzw. von Luft gesperrt ist, wobei ein weiterer Durchgang des Ventils (18) in diesem dritten Schaltzustand (56) geöffnet ist, wobei ein Strom an Luft oder an Insufflationsgas in ein Vorratsgefäß (118) an einer Spülflüssigkeit (120) führbar ist, und der weitere Durchgang des Ventiles mit einem Auslass des Vorratsgefäßes (118) verbunden ist, so dass im dritten Schaltzustand (56) des Ventiles (18) Spülflüssigkeit (120) durch das Instrument (12) von proximal nach distal führbar ist.

8. Insufflationssystem nach einem der Ansprüche 1 bis 7, **dadurch gekennzeichnet, dass** die Steuerung (100) eine Hystereseschaltung aufweist, die ein Umschalten des Umschaltventils (80) beim Übergang vom ersten Schaltzustand (52) in den zweiten Schaltzustand (54) bei einem vom Drucksensor (96) erfasstem Druckabfall verzögert.

9. Verfahren zum Betreiben eines Insufflationssystemes, mit einem Instrument (12) durch das ein Insufflationsgas führbar ist, und das ein Ventil (18) aufweist, das in einem ersten Schaltzustand ein Durchführen von gasförmigen Medium durch das Instrument mit einem niedrigen Druck freigibt und in einem zweiten Schaltzustand (54) ein Durchführen eines Insufflationsgases mit höherem Druck freigibt, und mit einem Insufflator (14), der ein Umschaltventil (80) zum alternativen Zuführen von Insufflationsgas (39) oder Luft (76) zum Instrument (12) aufweist, mit den Schritten:
Erfassen des Schaltzustandes des Ventiles (18) durch einen Drucksensor (96), der proximalseitig des Ventils (18) angeordnet ist,
Zuführen der erfassten Drucksignale zu einer Steuerung (100),
Schalten des Umschaltventiles (80) durch die Steuerung (100) auf Zufuhr von Insufflationsgas (39), wenn der zweite Schaltzustand des Ventiles (18) erfasst wird, und
Schalten des Umschaltventiles (80) in Zufuhr von Luft zum Ventil (18) wenn dessen erster Schaltzustand detektiert wird.

10. Verfahren nach Anspruch 9, mit dem Schritt
Zeitverzögern des Umschaltens des Umschaltventils (80) bei einem Übergang des Ventils (18) vom ersten Schaltzustand (52) in den zweiten Schaltzustand (54).

11. Verfahren nach Anspruch 9 oder 10, **dadurch gekennzeichnet, dass** die zuzuführende Luft im Insufflator komprimiert wird.

## Claims

1. An insufflation system comprising a medical instrument (12) having a channel (23) through which an insufflation gas (39) can be guided from a proximal end to a distal end thereof, wherein the instrument (12) comprises a valve (18) that is connected to the channel (23) and that, in a first switching state (52), leads the insufflation gas off to the environment and, in a second switching state (54), guides the insufflation gas through the instrument (12), and an insufflator (14) for providing and discharging the insufflation gas (39) to the channel (23), wherein a pressure sensor (96) is provided that is connected to the channel (23) and that is arranged to detect the two switching states (52, 54) of the valve (18), and comprising a control unit (100) that processes the pressure values detected by the pressure sensor (96), **characterized in that** a switch-over valve (80) is provided that is arranged fluidically upstream of the valve (18) and that, on the input side thereof, is arranged to be connected both to a source (64) of insufflation gas and also to a source (78) of air, and wherein the switch-over valve (80) is connected, on the output side thereof, to the channel (23), and **in that** the control unit (100) switches the switch-over valve (80) for connection to the source (78) of air (76) when the pressure sensor (96) detects the first switching state (52) of the valve (18), such that, in the first switching state (52), air instead of insufflation gas can be delivered to the instrument (12), and switches the switch-over valve (80) for connection to the source (64) of insufflation gas (39) when the pressure sensor (96) detects the second switching state (54), such that, in the second switching state (54), insufflation gas (39) instead of air (76) can be delivered to the instrument (12).

2. The insufflation system as claimed in claim 1, **characterized in that** the pressure sensor (96), the switch-over valve (80) and the control unit (100) are arranged in the insufflator (14).

3. The insufflation system as claimed in claim 1 or 2, **characterized in that** the source (78) of air comprises a compressor (74), that sucks in ambient air (76) and feeds the ambient air (76) to the channel (23) of the instrument (12).

4. The insufflation system as claimed in claim 3, **characterized in that** the compressor is arranged in the insufflator (14).

5. The insufflation system as claimed in one of claims 1 through 4, **characterized in that** the valve (18) of the instrument (12) comprises a branch line (28), that is open to the environment and that is connected to the channel (23).

6. The insufflation system as claimed in claim 5, **characterized in that** the branch line (28) comprises an outlet opening (50) to the environment, that is arranged to be closed by the finger (51) of a person handling the outlet opening (50).

7. The insufflation system as claimed in one of claims 1 through 6, **characterized in that** the valve (18) of the instrument (12) can be brought to a third switching state (56), in which insufflation gas or air is blocked from passing through, wherein a further passage of the valve (18) is opened in this third switching state (56), wherein a stream of air or of insufflation gas can be guided into a storage container (118) for a flushing liquid (120), and the further passage of the valve is connected to an outlet of the storage container (118), such that, in the third switching state (56) of the valve (18), flushing liquid (120) can be guided through the instrument (12) from the proximal end to the distal end.

8. The insufflation system as claimed in one of claims 1 through 7, **characterized in that** the control unit (100) comprises a hysteresis circuit that delays a switch-over of the switch-over valve (80) at the transition from the first switching state (52) to the second switching state (54) in the event of a pressure drop detected by the pressure sensor (96).

9. A method for operating an insufflation system comprising an instrument (12) through which an insufflation gas can be guided and comprising a valve (18) that, in a first switching state, allows gaseous medium to pass through the instrument at a low pressure and, in a second switching state (54), allows an insufflation gas to pass through at a higher pressure, and with an insufflator (14) comprising a switch-over valve (80) for alternately delivering insufflation gas (39) or air (76) to the instrument (12), said method comprising the steps of:
detecting the switching state of the valve (18) by means of a pressure sensor (96),
delivering the detected pressure signals to a control unit (100),
switching the switch-over valve (80), by means of the control unit (100), to enable delivery of insufflation gas (39) when the second switching state of the valve (18) is detected, and
switching the switch-over valve (80) to enable delivery of air to the valve (18) when the first switching state of the latter is detected.

10. The method as claimed in claim 9, comprising the step of
delaying the switch-over of the switch-over valve (80) at a change of the valve (18) from the first switching state (52) to the second switching state (54).

11. The method as claimed in claim 9 or 10, **characterized in that** the air to be delivered is compressed in the insufflator.

## Revendications

1. Système d'insufflation, comprenant un instrument médical (12) qui possède un canal (23), par le biais duquel un gaz à insuffler (39) peut être acheminé d'un côté proximal à un côté distal, l'instrument (12) possédant une valve (18) qui est en liaison avec le canal (23), laquelle, dans un premier état de commutation (52), provoque une évacuation du gaz à insuffler vers l'environnement et, dans un deuxième état de commutation (54), provoque un passage du gaz à insuffler à travers l'instrument (12), et comprenant un insufflateur (14) destiné à fournir et à délivrer le gaz à insuffler (39) au canal (23), un capteur de pression (96) relié au canal (23) étant présent, lequel permet de détecter les deux états de commutation (52, 54) de la valve (18), et comprenant une commande (100) qui traite les valeurs de pression détectées par le capteur de pression (96), **caractérisé en ce qu'**il existe une valve d'inversion (80) disposée avant la valve (18) du point de vue fluidique, dont l'entrée peut être reliée à la fois à une source (64) de gaz à insuffler qu'à une source (78) d'air, et la sortie de la valve d'inversion (80) étant reliée au canal (23) et la commande (100) commutant la valve d'inversion (80) sur la liaison avec la source (78) d'air (76) lorsque le capteur de pression (96) détecte le premier état de commutation (52) de la valve (18), de sorte que dans le premier état de commutation (52), de l'air peut être acheminé à l'instrument (12) au lieu du gaz à insuffler, et commutant la valve d'inversion (80) sur la liaison avec la source (64) de gaz à insuffler (39) lorsque le capteur de pression (96) détecte le deuxième état de commutation (54), de sorte que dans le deuxième état de commutation (54), du gaz à insuffler (39) peut être acheminé à l'instrument (12) au lieu de l'air (76).

2. Système d'insufflation selon la revendication 1, **caractérisé en ce que** le capteur de pression (96), la valve d'inversion (80) et la commande (100) sont disposés dans l'insufflateur (14).

3. Système d'insufflation selon la revendication 1 ou 2, **caractérisé en ce que** la source (78) d'air possède un compresseur (74) qui aspire l'air ambiant (76) et l'injecte dans le canal (23) de l'instrument (12).

4. Système d'insufflation selon la revendication 3, **caractérisé en ce que** le compresseur est disposé dans l'insufflateur (14).

5. Système d'insufflation selon l'une des revendications 1 à 4, **caractérisé en ce que** la valve (18) de l'instrument (12) possède une conduite de dérivation (28) ouverte vers l'environnement qui est reliée au canal (23).

6. Système d'insufflation selon la revendication 5, **caractérisé en ce que** la conduite de dérivation (28) possède une ouverture de sortie (50) vers l'environnement qui peut être fermée par le doigt (51) d'un opérateur.

7. Système d'insufflation selon l'une des revendications 1 à 6, **caractérisé en ce que** la valve (18) de l'instrument (12) peut être amenée dans un troisième état de commutation (56) dans lequel une traversée par le gaz à insuffler ou par l'air est bloquée, un passage supplémentaire de la valve (18) étant ouvert dans ce troisième état de commutation (56), un courant d'air ou de gaz à insuffler pouvant être amené dans une cuve de réserve (118) en un liquide de rinçage (120), et le passage supplémentaire de la valve est relié à une sortie de la cuve de réserve (118) de sorte que dans le troisième état de commutation (56) de la valve (18), du liquide de rinçage (120) peut être acheminé à travers l'instrument (12) du côté proximal au côté distal.

8. Système d'insufflation selon l'une des revendications 1 à 7, **caractérisé en ce que** la commande (100) possède un circuit à hystérésis qui retarde un basculement de la valve d'inversion (80) lors de la transition du premier état de commutation (52) dans le deuxième état de commutation (54) dans le cas d'une chute de pression détectée par le capteur de pression (96).

9. Procédé pour faire fonctionner un système d'insufflation, comprenant un instrument (12) à travers lequel peut passer un gaz à insuffler, et possédant une valve (18) qui, dans un premier état de commutation, libère un passage d'un fluide gazeux à travers l'instrument avec une faible pression et, dans un deuxième état de commutation (54), libère un passage d'un gaz à insuffler avec une haute pression, et comprenant un insufflateur (14) qui possède une valve d'inversion (80) destinée à l'acheminement alterné de gaz à insuffler (39) ou d'air (76) à l'instrument (12), comprenant les étapes suivantes :
détection de l'état de commutation de la valve (18) par un capteur de pression (96) qui est disposé du côté proximal de la valve (18),
acheminement des signaux de pression détectés à une commande (100),
commutation de la valve d'inversion (80) par la commande (100) en position d'acheminement de gaz à insuffler (39) lorsque le deuxième état de commutation de la valve (18) est détecté, et
commutation de la valve d'inversion (80) en position d'acheminement d'air à la valve (18) lorsque son premier état de commutation est détecté.

10. Procédé selon la revendication 9, comprenant l'étape suivante retardement du basculement de la valve d'inversion (80) lors d'une transition de la valve (18) du premier état de commutation (52) dans le deuxième état de commutation (54).

11. Procédé selon la revendication 9 ou 10, **caractérisé en ce que** l'air à acheminer est comprimé dans l'insufflateur.
